## Europäisches Patentamt
## European Patent Office
## Office européen des brevets

(11) Veröffentlichungsnummer: **0 121 491**
**B1**

(12) **EUROPÄISCHE PATENTSCHRIFT**

(45) Veröffentlichungstag der Patentschrift:
**17.09.86**

(21) Anmeldenummer: **84730016.7**

(22) Anmeldetag: **05.03.84**

(51) Int. Cl.⁴: **A 61 F 2/30**

(54) Implantat.

(30) Priorität: **04.03.83 DE 3308229**
**30.05.83 DE 3319916**

(43) Veröffentlichungstag der Anmeldung:
**10.10.84 Patentblatt 84/41**

(45) Bekanntmachung des Hinweises auf die Patenterteilung:
**17.09.86 Patentblatt 86/38**

(84) Benannte Vertragsstaaten:
**AT CH DE FR GB IT LI**

(56) Entgegenhaltungen:
**DE - A - 2 742 098**
**DE - U - 1 761 323**
**FR - A - 2 349 319**
**GB - A - 1 371 335**

(73) Patentinhaber: **MECRON MEDIZINISCHE PRODUKTE GMBH, Nunsdorfer Ring 25-27, D-1000 Berlin 48 (DE)**

(72) Erfinder: **Brinckmann, Paul, Hüfferstrasse 27, D-4400 Münster/Westf. (DE)**
Erfinder: **Anapliotis, Emmanuel, Tollensestrasse 16, D-1000 Berlin 37 (DE)**

(74) Vertreter: **Christiansen, Henning, Dipl.-Ing., Dietrich-Schäfer-Weg 21, D-1000 Berlin 41 (DE)**

ACTORUM AG

## Beschreibung

Die Erfindung betrifft ein Implantat nach dem Oberbegriff des Anspruchs 1.

Bei Implantaten und insbesondere Endoprothesen kann die Notwendigkeit bestehen, Implantatteile auszuwechseln oder neu zu verankern. Um beim Auswechseln eines (ersten) Teils die Explantation eines fest im Knochen einzementierten (zweiten) Teils der Prothese zu vermeiden, muss das erste Teil zum Auswechseln vom zweiten, im Knochen verbleibenden Teil getrennt werden. Dieses Trennen der ineinander verkeilten Teile erfordert grosse Kräft, und es besteht die Gefahr, dass infolge der Manipulation, beispielsweise infolge Abrtuschens von meisselartigen Werkzeugen, welche üblicherweise zum Trennen verwendet werden, Knochen- oder Weichteile beschädigt werden.

Derartige Implantate können vorzugsweise als Hüftgelenk- oder Knieprothese Verwendung finden. Dabei weist der zweite Teil neben einem Schaft einen konischen Zapfen auf, auf welchen das erste Teil aufsteckbar ist, an welchem sich eine künstliche Gelenkfläche befindet. Diese ist dem Verschleiss unterworfen, was ebenfalls dazu führen kann, dass das entsprechende Prothesenteil ausgewechselt werden muss. Im übrigen ist es auch dann, wenn nur die andere zugehörige Gelenkfläche bei einer Reoperation ausgewechselt werden muss, zweckmässig, von dem vorgeschlagenen Implantat ebenfalls das erste Teil mit der Gelenkfläche auszutauschen, da dies nur einen geringen Mehraufwand erfordert und eventuell eine später erforderlich werdende weitere Operation einspart. Es werden also bei einer Operation jeweils beide Gelenkflächen erneuert. Dabei braucht der operierende Chirurg auch nicht mehr darauf zu achten, dass während der Operation unbedingt Beschädigungen der am vorgeschlagenen Implantat befindlichen Gelenkflächen vermieden werden.

Der im Anspruch 1 angegebenen Erfindung liegt die Aufgabe zugrunde, ein Implantat der eingangs angegebenen Art zu schaffen, bei der sich die beiden Teile leichter voneinander trennen lassen; dabei soll insbesondere die Festigkeit und dauerhafte Haltbarkeit der Steckverbindung zwischen den beiden Teilen nicht beeinträchtigt werden.

Der Erfindung liegt die Erkenntnis zugrunde, dass ein Lösen von belastungsstabil miteinander verbundenen Prothesenteilen in besonders günstiger Weise mittels eines Elements möglich ist, das integraler Bestandteil des Implantats ist und mit diesem im Knochen befestigt wird.

Beim Zusammenfügen zweier die erfindungsgemässe Lösung aufweisender Prothesenteile wird vor dem Verbinden die Mutter über den Konus gestreift und bis zu einem Anschlag oder einer sonstigen Begrenzung auf das Gewinde aufgeschraubt, bevor die beiden Teile mit Hilfe des konischen Zapfens fest zusammengesteckt werden. Zum späteren Lösen dieser Steckverbindung wird die Mutter zurückgedreht, so dass sie das auf den konischen Zapfen gesteckte erste Teil von dem Konus abhebt.

Beim Zurückdrehen der Mutter muss das zweite Teil festgehalten werden. Es ist deshalb zweckmässig, es mit Angriffsflächen für ein Haltewerkzeug zur Drehmomentübertragung gegen Verdrehen um die Gewindeachse zu versehen.

Bei einer anderen Weiterbildung der Erfindung ist die Mutter mit einer umlaufenden Verzahnung, Nutung oder Lochung in der Weise versehen, dass ein in seiner Funktion einem Nutmutterschlüssel entsprechendes Werkzeug mit einem Stift, einer Nase oder einem Gegenzahn in die entsprechende Ausnehmung der Mutter eingreifen kann und ein Lösen bzw. Festziehen mit kurzen Winkelhüben ermöglicht. Damit ist eine Handhabung mit einem einfachen Werkzeug gegeben, wobei sich der Eingriffspunkt für den Stift oder Gegenzahn des Werkzeugs bevorzugt an der vom Bedienenden abgewandten Seite befindet, so dass die den Stift in Eingriff haltende Kraft durch die Einwirkung des als Maulschlüssel ausgebildeten Haltewerkzeugs kompensiert werden kann und insoweit ebenfalls das Entstehen von den festen Sitz des implantierten Schaftes lockernden Kräften verhindert ist. Wenn als Haltewerkzeug ein Nutmutterschlüssel verwendet wird, ist die Nase aus diesem Grunde — bezogen auf gleichgerichtete Schlüssel — gegenüber der entsprechenden Nase des anderen Schlüssels anzubringen, so dass sich die die Nasen in Eingriff haltenden Kräfte kompensieren.

Wird der Umfang der Mutter im Eingriffsbereich des Schlüssels — zur Aufnahme der entsprechenden Aussparungen und zur Verringerung der in der Aussparung zu übertragenden Kräfte beim Aufbringen des zum Lösen der Mutter erforderlichen Drehmoments — grösser gehalten und zur Gelenkkugel hin bis auf nahezu den zur Übertragung der axial gerichteten Kräfte auf die Gelenkkugel notwendigen kleinsten Querschnitt verringert, so resultiert daraus gleichzeitig der Vorteil, dass die Beweglichkeit der Gelenkschale gegenüber einem Konusende ohne Mutter kaum eingeschränkt ist.

Die Angriffsflächen für das das andere Teil festhaltende Werkzeug wird dabei bevorzugt in Form von Anfasungen am Prothesenschaft angebracht, da das Eingreifen der Gelenkkugel schwierig ist und zumindestens einen Satz auf die jeweilige Kugelgrösse abgestimmter Werkzeuge erfordert.

Gemäss einer bevorzugten Ausführung findet die Erfindung Anwendung bei einem Implantat zur Überbrückung grösserer Defekte im Bereich von Gelenken. Ein derartiges Implantat soll die Funktionen von Extremitäten, insbesondere des Beins erhalten, wenn in der Region des Gelenks (Hüft- bzw. Kniegelenks) bis zu einem Drittel der proximalen bzw. distalen Anteile des Femur und/oder proximalen Anteile der Tibia reseziert worden sind. Solche Resektionen können erforderlich werden bei primären Knochentumoren, Skelettmetastasen oder nach mehrfachem Austausch von künstlichem Kniegelenkersatz im Zusammenhang mit Infektionen. Dabei lassen sich mit Konus- bzw. Konusaufnahmeenden versehene Schaftteile unterschiedlicher Länge miteinander verbinden und — durch die Massnahmen gemäss der Erfindung — auch ohne besondere Schwierigkeiten wieder voneinander lösen.

Bei einer günstigen Ausführen als Kniegelenkersatz ist das Implantat nicht gelenkig ausgebildet; es soll eingesetzt werden, wo das Fehlen der muskulären Voraussetzungen die Anwendung eines (weiteren) künstlichen Kniegelenkersatzes nicht zulassen. Auch dabei erfolgt eine bevorzugte Ausgestaltung als modularer Implantatbausatz, bestehend aus einem in der Grösse abgestuften Satz von Implantaten, zur Einbringung in den Tibiaschaft. Die Femurimplantate tragen an einem Ende einen Innenkonus; die Tibiaimplantate einen Aussenkonus.

Die Masse bei der Fertigung von Implantatbausätzen sind bevorzugt einheitlich so gewählt, dass jedes Implantatteil jeder Länge mit jedem anderen Implantatteil jeder Länge kombiniert werden kann. Der Steckkonus sichert nach dem Zusammenstecken und einer einmaligen Belastung die rotationsstabile Verbindung der Teile. Das jeweils den Aussenkonus tragende Teil enthält unterhalb des Aussenkonus eine auf einem Gewinde laufende Mutter, die dazu benutzt werden kann, die Konussteckverbindung wieder zu lösen, falls das Implantat entfernt werden muss.

Insbesondere bei «kragenlosen» Hüftgelenkprothesen, bei denen also der Zapfen des zweiten Teils übergangslos in den Schaft übergeht, hat sich nun gezeigt, dass das vorgeschlagene Implantat schlecht anwendbar ist, da sich zwischen dem Schaft und der aufgeschraubten Mutter [mit der das erste Teil (künstliche Gelenkkugel) vom konischen Schaft des zweiten Teils abgehoben werden kann] schlecht Angriffsflächen für ein Haltewerkzeug gegen Verdrehen um die Gewindeachse anbringen lassen. Es hat sich auch gezeigt, dass das Festhalten des ersten Teils (Gelenkkugel) beim Drehen der Mutter schlecht durchführbar ist: Wegen unterschiedlicher Kugeldurchmesser müssten unterschiedliche Werkzeuge bereitgehalten werden, die zudem insbesondere bei grösseren Kugeldurchmessern unhandlich sind. Sie greifen auch schlecht an der glatten Kugeloberfläche, so dass immer die Gefahr besteht, dass bei schlechtem Sitz des Werkzeugs Späne entstehen, die in die Operationswunde fallen und zu Komplikationen führen können. Ferner hat sich gezeigt, dass die Mutter beim vorgeschlagenen Implantat die Beweglichkeit des künstlichen Gelenks einschränken kann.

Mit der Erfindung ist erreicht worden, dass sich das erste Teil vom zweiten stets mit Hilfe einer zwischen Schaft und Konus auf dem zweiten Teil angeordneten Mutter lösen lässt. Wenn es bei Implantatteilen — insbesondere bei «kragenlosen» Hüftgelenkprothesen, bei denen der Zapfen des zweiten Teils ohne ausgeprägte Kanten in den Schaft übergeht — unerwünscht ist, dass hier Anfasungen zum Festhalten durch ein Werkzeug angebracht werden, so wird gemäss einer vorteilhaften Weiterbildung zwischen Konusende und Schaft zwischen der Mutter und dem ersten Teil (z.B. Gelenkkugel) ein Zwischenraum vorgesehen. In diesem Zwischenraum befinden sich am zweiten Teil Angriffsflächen für ein Haltewerkzeug, mit welchem das Verdrehen des Implantats beim Drehen der Mutter verhindert werden kann, so dass das Auftreten von den festen Sitz des implantierten Prothesenschafts beeinträchtigenden Kräften verhindert ist.

Zum Lösen des ersten Teils vom zweiten Teil des Implantats wird also das Haltewerkzeug im Bereich der Angriffsflächen angesetzt, die Mutter in Richtung auf das Haltewerkzeug gedreht und damit das Haltewerkzeug gegen das erste Teil gedrückt. Beim weiteren Drehen der Mutter verschiebt sich das Haltewerkzeug auf den Angriffsflächen am ersten Teil des Implantats. Das Haltewerkzeug dient dabei als Distanzstück zwischen Mutter und erstem Teil. Es überträgt die Kraft von der Mutter auf das erste Teil, da es auf diese Weise vom Konus des zweiten Teils abgehoben wird.

Die Tatsache, dass sich im implantierten Zustand zwischen dem ersten Teil und der Mutter nun ein Zwischenraum mit den Angriffsflächen befindet, ermöglicht eine grössere Beweglichkeit für ein benachbartes Gelenk, das beispielsweise aus dem Gelenkkopf und einer künstlichen Hüftpfanne besteht.

Vorteilhafte Weiterbildungen der Erfindung sind in den Unteransprüchen gekennzeichnet bzw. werden bei der nachstehenden Darstellung zusammen mit bevorzugten Ausführungsbeispielen der Erfindung näher beschrieben. Es zeigen:

Fig. 1 ein erstes vorteilhaftes Ausführungsbeispiel in Form einer Hüftgelenkprothese und

Fig. 2 das Ausführungsbeispiel gemäss Fig. 1 im Schnitt mit zugehörigem Betätigungswerkzeug,

Fig. 3 ein Implantat zur Überbrückung knöcherner Defekte im Bereich des Kniegelenks,

Fig. 4 zeigt ein zweiteres Ausführungsbeispiel einer Teilekombination in Form einer Hüftgelenkprothese mit zugehörigem Haltewerkzeug,

Fig. 5 ein Schnittbild entlang der Linie V-V in Fig. 4,

Fig. 6 einen Ausschnitt aus Figur 4, modifiziert entsprechend einer anderen Ausführungsvariante, und

Fig. 7 das zugehörige Haltewerkzeug als Schnitt entlang der Linie VII in Fig. 6.

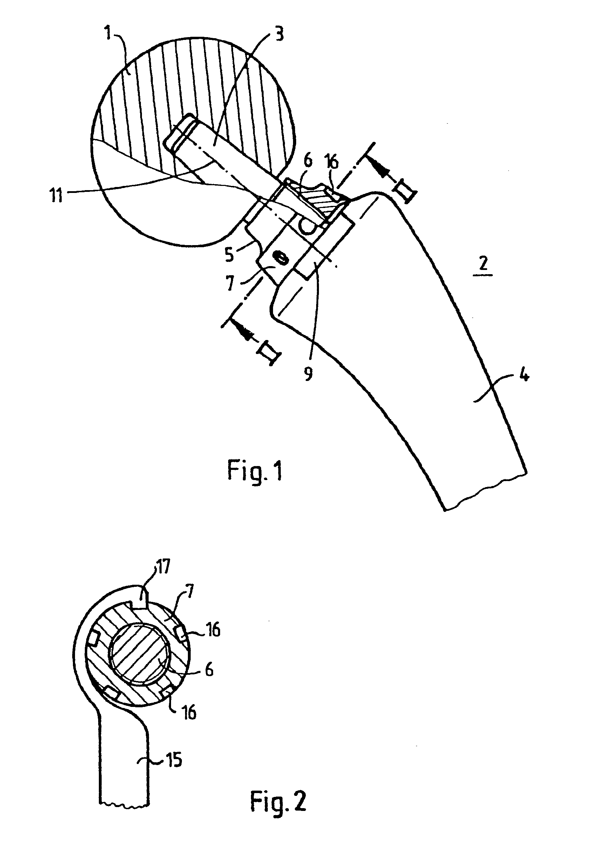

In Fig. 1 ist schematisch der Kopfbereich einer künstlichen Hüftgelenkprothese mit einem im Schnitt gezeigten ersten Teil 1 mit Kugelgelenkfläche wiedergegeben. Der rechte Teil des Kopfbereichs ist dabei geschnitten dargestellt. Dieses erste Teil 1 ist auf einen konischen Zapfen 3 eines zweiten Teils 2 aufgesteckt, das einen Schaft 4 aufweist. Zwischen dem Fuss des Zapfens 3 und dessen konischem freien Ende befindet sich ein Gewinde 6, auf welches eine Mutter 7 aufgeschraubt ist. Diese weist in dem dargestellten Zustand, also im implantierten Zustand der Prothese, einen (gegebenenfalls geringfügigen) Abstand zum ersten Teil 1 der Prothese auf.

Soll nun dieser erste Teil 1 vom zweiten Teil 2 gelöst werden, so wird die Mutter 7 in Richtung auf das freie Ende des Zapfens 3 geschraubt, und zwar so lange, bis sich das erste Teil 1 vom Zapfen 3 löst.

Beim Drehen der Mutter 7 muss das zweite Teil 2 festgehalten werden. Dieses weist zu diesem Zweck Angriffsflächen 9 in Form von symmetrischen seitlichen Anfasungen auf für ein Haltewerkzeug, beispielsweise einen Schraubenschlüssel, mit dessen Hilfe die Verdrehung des Schafts 4 um die Gewindeachse verhindert werden kann. Die gegenüberliegende Anfasung liegt bei der Darstellung in der Zeichnung unten und ist nicht sichtbar.

Es werden nur relativ geringe Kräfte zum Lösen der beiden Teile 1 und 2 voneinander benötigt, weil das Gewinde 6 in Verbindung mit der Mutter 7 nach Art eines Schneckentriebes für eine starke Kraftübersetzung bezüglich der an der Mutter angreifenden Drehkräfte für die zum Lösen der Teile erforderlichen Axialkräfte sorgt.

In Fig. 2 ist die Mutter 7 in Draufsicht mit zugehörigem Betätigungswerkzeug 15 dargestellt, wobei die Mutter eine im wesentlichen zylinder- oder kegelmantelförmige Aussenkontur aufweist, in die lochförmige Aussparungen 16 in Umfangsrichtung regelmässig verteilt eingelassen sind.

In diese Aussparungen 16 greift eine als Gegenzahn ausgebildete Nase 17 ein, so dass ein Anziehen bzw. Lösen der Mutter 7 mit kleinen Winkelhüben möglich ist, wenn in jeweils aufeinanderfolgende Aussparungen 16 eingegriffen wird. Die Nase ist an die Form der Aussparungen angepasst — hat also bei einer Aussparung in Form einer Sackbohrung die Gestalt eines zylindrischen Stifts. Die Aussparungen 16 können — je nach Bedarf — mit grösseren oder kleineren Abständen in Umfangsrichtung verteilt angeordnet sein. Entsprechend können die Aussparungen auch mit polygonem Querschnitt ausgebildet sein.

Der Angriffspunkt des Gegenzahns, der Nase oder des Stifts 17 befindet sich an der vom Bedienenden abgewandten Seite des Schafts am Werkzeug 15, so dass die beim Festziehen oder Lösen der Mutter 7 auftretenden Kräfte beim Gegenhalten unter Benutzung des Maulschlüssels 10 weitgehend kompensiert werden, da mit diesem Werkzeug beim Gegenhalten eine Druckkraft ausgeübt werden muss, während der Eingriff des Gegenzahns oder Stifts 17 eine Zugkomponente erfordert. Bei anders ausgebildeten Haltewerkzeugen wird durch eine entsprechend bei Benutzung einander gegenüberliegende Anordnung der in den Schaft eingreifenden Nasen ebenfalls für eine Kraftkompensation gesorgt.

Durch die Ausbildung der Mutter 7 mit zylinder- oder kegelmantelförmiger Aussenfläche und darin befindlichen Aussparungen 16 lässt sich eine Formgestaltung erreichen, welche bezüglich ihrer Aussenkontur — mit Ausnahme der Aussparungen 16 — derjenigen des Übergangsbereichs des Prothesenschafts 4 zum Konus 3 hin weitgehend angepasst ist.

Bevorzugt ist bei einer eine Gelenkkugel 1 aufweisenden Endoprothese der dem Konus 3 benachbarte Durchmesserbereich 5, wie es aus Fig. 1 ersichtlich ist, möglichst schlank gehalten, so dass der Bewegungsbereich der zugehörigen Gelenkkapsel (in der Zeichnung nicht dargestellt) nur wenig eingeschränkt ist. Die Aussparungen 16 befinden sich an der der Gelenkkugel 1 abgewandten Seite. Damit ist auch ein optimaler Konturübergang zum Schaft 4 gewährleistet, wobei die Länge der Mutter gegebenenfalls verschiedenen Halslängen anpassbar ist.

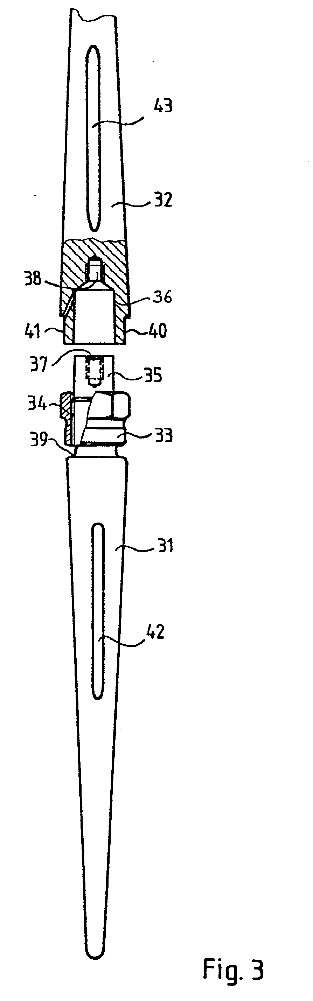

In Fig. 3 ist ein Implantat zur Überbrückung grösserer knöcherner Defekte im Bereich des Kniegelenks wiedergegeben. Es besteht aus einem zweiten Teil 31 zur Verankerung in der Tibia und einem ersten Teil 32 zur Verankerung im Femur. Die Funktion und Anordnung einer Mutter 33, die auf einem Gewindeaufsatz 34 eines Konus 35 aufgeschraubt ist, entspricht hinsichtlich ihrer Funktion dem in Fig. 1 dargestellten Ausführungsbeispiel. Das Gewinde 34 ist erhaben ausgebildet, so dass die Mutter 33 über den Konus hinweg aufgeschraubt werden kann. Im Femurteil 32 findet sich eine Aussparung 36 zur Aufnahme des am Tibiateil 31 angeordneten Konus 35. Beide Teile weisen noch zusätzliche Sackbohrungen 37 bzw. 38 mit Gewinde auf, welche zur Aufnahme eines Werkzeugs zum Halten oder Ausziehen des betreffenden Teils dienen können.

Die in Fig. 3 wiedergegebene Anordnung zeigt die beiden Implantatteile in der Position kurz nach dem Lösen, wobei die Mutter 33 in einer Position in geringer Entfernung vom Anschlag 39 dargestellt ist, gegen den sie im implantierten Zustand der Anordnung geschraubt ist. Zum Gegenhalten während des Lösens der Mutter mit einem Schraubenschlüssel dienen Anfasungen 40 und 41 am Femurteil. Diese Anordnung ist an und für sich überraschend, da nach den mechanischen Gesetzen an sich an dem Teil 31 festgehalten und ein Gegenmoment erzeugt werden müsste. Da dieser Schaftteil aber nur schwer zugänglich ist, kann bis zum Lösen der Verbindung — und dazu dient die Kraftaufbringung — entsprechend am Femurteil 32 gegengehalten werden.

Beim Einbau der erfindungsgemässen Implantate wird zweckmässigerweise wie folgt vorgegangen:

Die Implantate werden aus dem Baukasten je nach Höhe der an Femur und Tibia erfolgten Resektion ausgewählt. Die Auswahl erfolgt dabei nach dem Gesichtspunkt, dass die Implantate noch sicher genug im Knochen verankert werden können und dass die Beinlänge der Länge des gesunden Beins so gut wie möglich angeglichen werden soll. Die Verankerung im Knochen erfolgt mit Hilfe von Knochenzement, analog dem Vorgehen bei künstlichem Gelenkersatz. Die Implantate haben eine glatte, konische Form; jedoch weisen sie zusätzlich je eine in Längsrichtung eingefräste Nut 42 bzw. 43 auf, die die Rotationsstabilität im Zementlager sichern. Auch eine zementfreie Implantation ist möglich. Hierbei wäre statt dessen eine andere (strukturierte) Oberflächengestaltung vorzusehen.

Die Einbauinstrumente umfassen einen Satz von Probierteilen, die die Auswahl aus dem modularen System vereinfachen, zwei Schlüssel, die das Lösen eines zusammengesteckten Konus ermöglichen und einen Haken zum Ausziehen der Implantate (die Implantate und die Probierteile enthalten an ihrer Stirn dazu die zuvor erwähnten Innengewinde 37 und 38).

Als Material für die Implantate kommt jede implantierfähige Metallegierung in Frage; aus Gewichtsgründen ist eine Titanlegierung, wie beispielsweise TiAlV64 günstig.

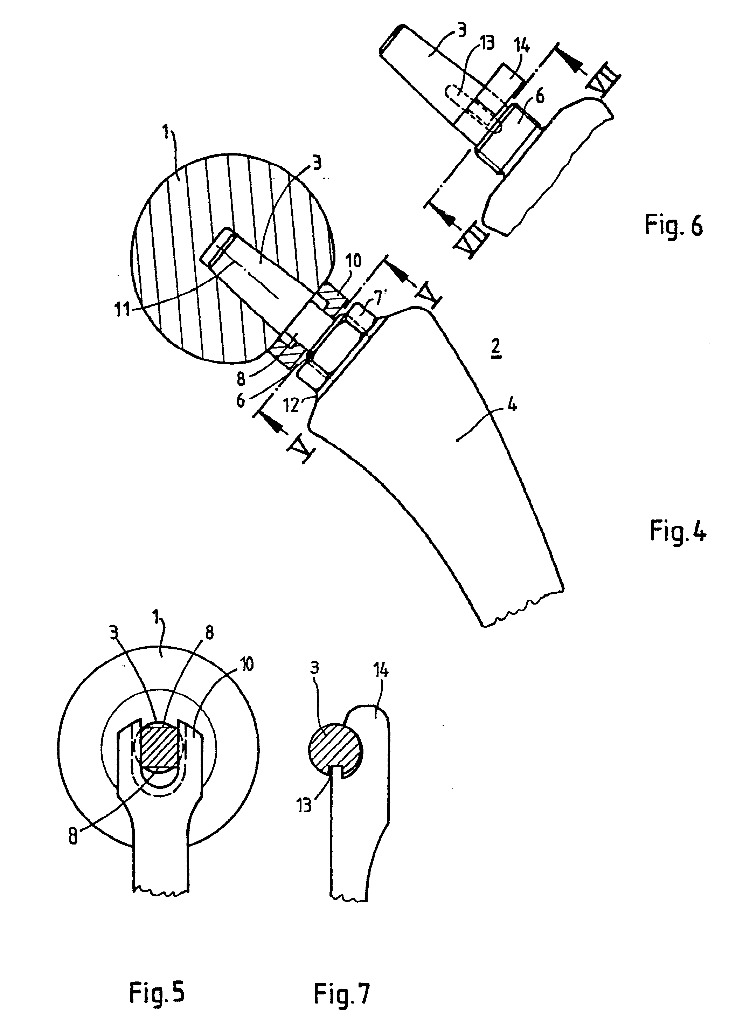

In Fig. 4 ist schematisch ein Hüftgelenkskopf mit einem im Schnitt gezeugten ersten Teil 1 mit einer Gelenkkugel wiedergegeben. Dieses erste Teil 1 ist auf einen konischen Zapfen 3 eines zweiten Teils 2 aufgesteckt, das mit einem Schaft 4 verbunden ist. Zwischen dem Konus 3 und dem Schaft 4 befindet sich ein Gewinde 6, auf das eine Mutter 7' aufgeschraubt ist. Diese weist in der dargestellten Position, die dem implantierten Zustand entspricht, einen merklichen Abstand zum ersten Teil 1 des Implantats auf. (Wie ersichtlich entsprechen die mit übereinstimmenden Bezugzeichen versehenen Positionen im wesentlichen der Darstellung gemäss Fig. 1.)

In dem Zwischenraum zwischen dem Konus 3 und der Mutter 7' befindet sich am zweiten Teil 2 ein Implantatabschnitt mit Angriffsflächen für ein Haltewerkzeug, von denen eine ebene Angriffsfläche 8 dem Betrachter zugewandt ist. Wie entsprechend dem Schnitt V-V in Fig. 5 zu sehen ist, handelt es sich bei der Angriffsfläche 8 um eine von vier Angriffsflächen eines Vierkants. Zwei weitere einander parallel gegenüberliegende Angriffsflächen sind ebenfalls mit 8 bezeichnet. An den beiden anderen Angriffsflächen des Vierkants greift gemäss der Darstellung ein Maulschlüssel 10 an, der zum Festhalten des zweiten Teils 2 gegen Verdrehen um die Längsachse 11 dient, aber zugleich auch Distanzstück bildet zwischen Mutter 7' und Gelenkkugel 1.

Soll nun das erste Teil 1 vom zweiten Teil 2 gelöst werden, so wird die Mutter 7' in Richtung auf den Konus 3 geschraubt, und zwar zunächst so lange, bis sie den Maulschlüssel 10 berührt. Beim Weiterdrehen der Mutter 7' wird der Maulschlüssel 10 gegen eine Anlagefläche von Teil 1 gedrückt und hebt dieses langsam vom Konus 3 ab. Der Maulschlüssel 10 wird beim Abheben des Teils 1 in Richtung der Achse 11 bewegt, bleibt dabei aber mit den Angriffsflächen, die zwischen dem Gewinde 6 und dem Konus 3 angeordnet sind, in Eingriff.

Gemäss Fig. 4 hat der Maulschlüssel an seiner Unterseite eine Schlüsselweite entsprechend den Abmessungen des durch die Angriffsflächen am Teil 2 gebildeten Vierkants. Die Schlüsselweite ist aber an der Oberseite des Maulschlüssels 10 erweitert, damit der Maulschlüssel nicht an dem aus dem Teil 1 herausragenden Konus hängenbleibt.

Die Mutter 7' weist eine weitgehend abgerundete und damit dem Teil 2 angepasste Kontur auf. An ihrer Unterseite in Fig. 1 befindet sich ein ringförmiger Ansatz 12, der im implantierten Zustand auf einem Absatz am Schaft 4 aufliegt, so dass eine Abdichtung des Gewindes 6 gegen eindringende Körperflüssigkeit gegeben ist.

Bei dem Detailausschnitt nach Fig. 6 ist anstelle eines Vierkants in dem Implantatabschnitt zwischen dem Konus 3 und dem Gewinde 6 eine Längsnut 13 vorgesehen.

Fig. 7 zeigt, wie in diese Längsnut 13 anstelle eines Maulschlüssels ein Nutmutterschlüssel 14 eingreifen kann, dessen Stärke wiederum so zu wählen ist, dass er beim Abheben des Teils 1 die Entfernung zwischen diesem Teil und der Mutter 7' überbrückt. Die Nut 13 reicht bis in den Konus hinein, damit sich der Nutmutterschlüssel beim Abheben des Teils 1 vom Konus 3 ohne Behinderung weit genug in Richtung auf den Konus 3 bewegen kann.

Die Erfindung beschränkt sich in ihrer Ausführung nicht auf das vorstehend angegebene Beispiel. Vielmehr sind eine Vielzahl von im Bereich der Patentansprüche liegenden Varianten denkbar, welche von der dargestellten Lösung auch bei grundsätzlich anders gearteten Ausführungen Gebrauch machen.

**Patentansprüche**

1. Implantat, bestehend aus mindestens zwei Teilen, von denen das erste (1, 32) auf einen konischen Zapfen (3, 35) am zweiten Teil (2, 31) steckbar ist, dadurch gekennzeichnet, dass der Zapfen (3, 35) im Bereich des sich erweiternden Konusendes ein Gewinde (6, 34) mit einer Mutter (7, 7', 33) aufweist, die in Richtung auf das erste aufgesteckte Teil (1, 32) in der Weise schraubbar ist, dass es sich vom Zapfen (3, 35) löst.

2. Implantat nach Anspruch 1, dadurch gekennzeichnet, dass das erste Teil (1, 32) und/oder zweite Teil (2, 31) Angriffsflächen (8, 9, 40, 41) für ein Haltewerkzeug zur Übertragung eines Drehmoments gegen Verdrehen um die Gewindeachse aufweist.

3. Implantat nach einem der vorangehenden Ansprüche, dadurch gekennzeichnet, dass das den Zapfen (3, 35) oder die Aufnahme (36) für den Zapfen tragende Element mit einem in einen Knochen einsetzbaren Schaftteil (4, 31, 32) verbunden ist.

4. Implantat nach Anspruch 3, dadurch gekennzeichnet, dass das den Zapfen (3) tragende Element den Schaftteil (2) und das die Aufnahme für den Zapfen tragende Element den Gelenkteil (1) für eine Hüftgelenksprothese bildet.

5. Implantat nach Anspruch 3, dadurch gekennzeichnet, dass zur Überbrückung grösserer knöcherner Defekte, insbesondere im Bereich des Kniegelenks, die beiden Teile (31, 32) als starr miteinander verbindbare Schaftteile ausgebildet sind.

6. Implantat nach einem der vorangehenden Ansprüche, dadurch gekennzeichnet, dass eine Anzahl von Teilen unterschiedlicher Länge mit einheitlichen Anschlussstücken einen Bausatz bilden.

7. Implantat nach einem der vorangehenden Ansprüche, dadurch gekennzeichnet, dass die Mutter (7, 7') als Angriffsflächen für ein Werkzeug an ihrer Umfangsfläche in regelmässigen Abständen Aussparungen (16) mit Flächen enthält, die durch in Umfangsrichtung weisende Vektoren festgelegt sind.

8. Implantat nach Anspruch 7, dadurch gekennzeichnet, dass es sich bei den Aussparungen (16) um Teile einer Verzahnung, um Nuten oder um Sackbohrungen mit rundem bzw. polygonalem Querschnitt handelt.

9. Implantat nach einem der vorangehenden Ansprüche, dadurch gekennzeichnet, dass die Mutter (7, 7', 33) der übrigen Implantatkontur weitgehend angepasst ist.

10. Implantat nach einem der vorangehenden Ansprüche, dadurch gekennzeichnet, dass die Mutter (7') an demjenigen Ende, welches vom Zapfen (3) abgewandt ist, einen das Gewinde dichtenden, ringförmigen Ansatz (12) aufweist.

11. Implantat nach einem der vorangehenden Ansprüche zur Anwendung für eine Gelenkprothese mit einer auf den Zapfen (3) aufsteckbaren Gelenkkugel (1), dadurch gekennzeichnet, dass die Mutter (7) im Bereich ihrer Angriffsflächen (16) für ein Werkzeug einen grösseren Durchmesser aufweist als derjenige Bereich (5), welcher der Gelenkkugel (1) benachbart ist.

12. Implantat nach einem der vorangehenden Ansprüche, dadurch gekennzeichnet, dass die Angriffsflächen (8) des ersten und/oder zweiten Teils näher am Zapfen (3) angeordnet sind als die vom Zapfen am weitesten entfernten Teile des Gewindes (6) und dass die Angriffsflächen (8) derart ausgebildet sind, dass sie mit Hilfe der Mutter (7') eine Verschiebung eines in Eingriff befindlichen Haltewerkzeugs (10) in Axialrichtung des Gewindes (6) in Richtung auf das aufgesteckte erste Teil (1) bis zu dessen Ablösen vom zweiten Teil (2) erlauben.

13. Implantat nach Anspruch 12, dadurch gekennzeichnet, dass eine Angriffsfläche (8) eben und Teil eines zylinder- und/oder prismenförmigen Implantatabschnitts ist, dessen Achse (11) im wesentlichen parallel zur Gewinde- und/oder Zapfenachse verläuft.

14. Implantat nach Anspruch 12, dadurch gekennzeichnet, dass die Angriffsflächen (8) parallel zueinander an einander gegenüberliegenden Seiten des Implantats verlaufen.

15. Implantat nach Anspruch 12, dadurch gekennzeichnet, dass als eine Angriffsfläche die Seitenwand einer Längsnut (13) vorgesehen ist.

## Claims

1. An implant consisting of at least two parts, of which the first (1, 32) can be fitted on a tapered pin (3, 35) on the second part (2, 31), characterised in that, in the region of the widening end of the taper, the pin (3, 35) has a thread (6, 34) with a nut (7, 7', 33) which can be screwed in the direction of the first fitted-on part (1, 32) in such a manner that it besomes detached from the pin (3, 35).

2. An implant as claimed in Claim 1, characterised in that the first (1, 32) and/or second part (2, 31) has engagement faces (8, 9, 40, 41) for a holding tool to transmit a torque against turning about the axis of the thread.

3. An implant as claimed in any of the preceding Claims, characterised in that the element carrying the pin (3, 35) or the receiver (36) for the pin is connected to a shank member (4, 31, 32) which can be inserted in a bone.

4. An implant as claimed in Claim 3, characterised in that the element carrying the pin (3) forms the shank member (2) and the element carrying the receiver for the pin forms the joint member (1) for a hip-joint prosthesis.

5. An implant as claimed in Claim 3, characterised in that in order to bridge major bone defects, particularly in the region of the knee joint, the two parts (31, 32) are constructed in the form of shank members which can be ridigly connected to one another.

6. An implant as claimed in any of the preceding Claims, characterised in that a plurality of parts of different length with uniform connecting pieces form an assembly kit.

7. An implant as claimed in any of the preceding Claims, characterised in that the nut (7, 7') incorporates at its circumferential surface recesses (16) which are regularly spaced and have faces which are determined by vectors facing in the circumferential direction as engagement faces for a tool.

8. An implant as claimed in Claim 7, characterised in that the recesses (16) are parts of toothing or are grooves or blind bores with a round or polygonal cross-section.

9. An implant as claimed in any of the preceding Claims, characterised in that the nut (7, 7', 33) is largely adapted to the contour of the rest of the implant.

10. An implant as claimed in any of the preceding Claims, characterised in that the nut (7') comprises, at the end which is remote from the pin (3), an annular shoulder (12) sealing the thread.

11. An implant as claimed in any of the preceding Claims to be used for a joint prosthesis having a spherical part (1) which can be fitted on the pin (3), characterised in that, in the region of its engagement faces (16) for a tool, the nut (7) has a larger diameter than the region (5) which is adjacent to the spherical part (1) of the joint.

12. An implant as claimed in any of the preceding Claims, characterised in that the engagement faces (8) of the first and/or second part are disposed closer to the pin (3) than the parts of the thread (6) furthest away from the pin and that the engagement faces (8) are so constructed that, with the aid of the nut (7'), they permit a displacement of a holding tool (10), which is in engagement, in the axial direction of the thread (6) towards the fitted-on first part (1) until it becomes separated from the second part.

13. An implant as claimed in Claim 12, characterised in that one engagement face (8) is plane and is part of a cylindrical and/or prismatic implant portion, the axis (11) of which extends substantially parallel to the axis of the thread and/or of the pin.

14. An implant as claimed in Claim 12, characterised in that the engagement faces (8) extend parallel to one another at opposite sides of the implant.

15. An implant as claimed in Claim 12, characterised in that the side wall of a longitudinal groove (13) is provided as an engagement face.

## Revendications

1. Implant, constitué d'au moins deux parties, dont la première (1, 32) peut s'enficher sur un tenon conique (3, 35) de la seconde partie (2, 31), caractérisé en ce que le tenon (3, 35) présente, dans la zone de l'extrémité du cône qui va en s'élargissant, un filetage (6, 34) avec un écrou (7, 7', 33) que l'on peut visser en direction de la première partie enfichée (1, 32) de façon qu'elle se sépare du tenon (3, 35).

2. Implant selon la revendication 1, caractérisé en ce que la première (1, 32) et/ou la seconde (2, 31) partie présente des surfaces de prise (8, 9, 40, 41) pour un outil de maintien pour transmettre un moment de rotation opposé à une rotation autour de l'axe du filetage.

3. Implant selon l'une des revendications précédentes, caractérisé en ce que l'élément qui porte le tenon (3, 35) ou le logement (36) pour le tenon est relié à une partie formant fût (4, 31, 32) que l'on peut insérer dans un os.

4. Implant selon la revendication 3, caractérisé en ce que l'élément qui porte le tenon (3) constitue la partie formant fût (2), et l'élément qui porte le logement pour le tenon, la partie articulée (1), pour une prothèse d'articulation de la hanche.

5. Implant selon la revendication 3, caractérisé en ce que pour ponter des déficiences osseuses plus importantes, en particulier dans la zone de l'articulation du genou, les deux parties (31, 32) sont conçues sous forme de parties formant fût que l'on peut relier rigidement l'une à l'autre.

6. Implant selon l'une des revendications précédentes, caractérisé en ce qu'un certain nombre de pièces de différentes longueurs présentant des raccords homogènes constituent un ensemble.

7. Implant selon l'une des revendications précédentes, caractérisé en ce que l'écrou (7, 7') contient, comme surface de prise pour un outil, sur sa surface périphérique, des évidements (16) à distance régulière avec des surfaces qui sont définies par des vecteurs dirigés dans la direction périphérique.

8. Implant selon la revendication 7, caractérisé en ce que, dans le cas des évidements (16), il s'agit de parties d'une denture, de rainures ou d'alésages borgnes à section ronde ou polygonale.

9. Implant selon l'une des revendications précédentes, caractérisé en ce que l'écrou (7, 7', 33) est largement adapté au reste du contour de l'implant.

10. Implant selon l'une des revendications précédentes, caractérisé en ce que l'écrou (7') présente, à son extrémité opposée au tenon (3), un prolongement annulaire (12) qui assure l'étanchéité du filetage.

11. Implant selon l'une des revendications précédentes, pour emploi comme prothèse d'articulation avec une shpère d'articulation (1) enfichable sur le tenon (3), caractérisé en ce que l'écrou (7) présenté, dans la zone de ses surfaces de prise (6) pour un outil, un plus grand diamètre que celui de la zone (5) voisine de la sphère d'articulation (1).

12. Implant selon l'une des revendications précédentes, caractérisé en ce que les surfaces de prise (8) de la première partie et/ou de la seconde partie sont disposées plus près du tenon (3) que les parties du filetage (6) qui sont le plus éloignées du tenon; et en ce que les surfaces de prise (8) ont une forme telle qu'elles permettant, à l'aide de l'écrou (7), un coulissement d'un outil de maintien (10), qui se trouve en prise, dans la direction axiale du filetage (6), en direction de la première partie (1) enfichée, jusqu'à sa séparation d'avec la seconde partie (2).

13. Implant selon la revendication 12, caractérisé en ce qu'une surface de prise (8) est plate et fait partie d'une portion de l'implant de forme cylindrique et/ou prismatique dont l'axe (11) court essentiellement parallèlement à l'axe du filetage et/ou du tenon.

14. Implant selon la revendication 12, caractérisé en ce que les surfaces de prise (8) courent parallèlement l'une à l'autre sur les faces de l'implant opposées l'une à l'autre.

15. Implant selon la revendication 12, caractérisé en ce que comme surface de prise est prévue la paroi latérale d'une rainure longitudinale (13).

Fig. 1

Fig. 2

**Fig. 3**

Fig. 6

Fig. 4

Fig. 5

Fig. 7